# EUROPÄISCHE PATENTSCHRIFT

(11) **EP 3 861 049 B1**
(45) Veröffentlichungstag und Bekanntmachung des Hinweises auf die Patenterteilung: **11.12.2024**
(21) Anmeldenummer: 19774118.4
(22) Anmeldetag: 01.10.2019
(51) Int. Cl.: C08G 59/50, C09D 163/00

(54) **HÄRTER FÜR EPOXIDHARZE**
HARDENER FOR EPOXY RESINS
DURCISSEUR POUR ADHÉSIFS À BASE DE RÉSINE ÉPOXY

(30) Priorität: 01.10.2018 EP 18197992
(43) Veröffentlichungstag der Anmeldung: 11.08.2021
(60) Teilanmeldung: 24196102.8
(73) Patentinhaber: Sika Technology AG, 6340 Baar (CH)
(72) Erfinder: KASEMI, Edis, 8046 Zürich (CH); KRAMER, Andreas, 8008 Zürich (CH); STADELMANN, Ursula, 8046 Zürich (CH); BURCKHARDT, Urs, 8049 Zürich (CH)
(74) Vertreter: Sika Patent Attorneys
(86) Internationale Anmeldenummer: PCT/EP2019/076563
(87) Internationale Veröffentlichungsnummer: WO 2020/070112

(56) Entgegenhaltungen:
- EP-A1- 3 375 802
- DE-A1- 2 853 752
- US-A- 2 739 981

## Beschreibung

### Technisches Gebiet

Härter für Epoxidharze, Epoxidharz-Zusammensetzungen und ihre Verwendung, insbesondere als Beschichtung.

### Stand der Technik

Beschichtungen auf Epoxidharz-Basis sind im Bauwesen weit verbreitet. Sie bestehen aus flüssigen Harz- und Härterkomponenten, welche vor der Applikation gemischt werden und dann bei Umgebungstemperaturen im Bereich von etwa 5 bis 35°C zu einem hochfesten und beständigen Material aushärten. Solche Epoxidharz-Beschichtungen haben eine Neigung zu Oberflächenstörungen wie Trübungen, Flecken, Rauheit oder Klebrigkeit, was auch als "Blushing" bezeichnet wird. Blushing wird durch die Salzbildung der in der Härterkomponente enthaltenen Amine mit Kohlendioxid (CO₂) aus der Luft verursacht und tritt besonders bei hoher Luftfeuchtigkeit und tiefen Temperaturen auf. Insbesondere in ästhetisch anspruchsvollen Beschichtungsanwendungen wie Bodenbelägen ist das Auftreten von Blushing-bedingten Oberflächenstörungen äusserst unvorteilhaft und erfordert meist ein aufwendiges Nachbearbeiten bzw. Überbeschichten der schadhaften Stellen oder häufig sogar der ganzen Beschichtung.

Mittels Verdünnern wird die Viskosität einer Epoxidharz-Zusammensetzung gesenkt, damit sie einfach applizierbar ist und die Substratoberflächen gut benetzt. Gleichzeitig vermindern Verdünner auch die Anfälligkeit auf Blushing. Die üblichen Verdünner, wie beispielsweise Benzylalkohol, sind flüchtige Verbindungen (VOC oder SVOC), welche bei der Aushärtung nicht in die Polymermatrix eingebaut werden und somit zu Emissionen führen können. Für emissionsarme Produkte, wie sie von Verbrauchern zunehmend nachgefragt werden, können Verdünner deshalb nur in geringer Menge oder gar nicht verwendet werden.

Zur Verminderung von Blushing und als Reaktivverdünner können in der Härterkomponente alkylierte Amine eingesetzt werden, wie in EP 2,943,464, WO 2016/023839, EP 3,138,863 oder EP 3,144,335 beschrieben. Besonders geeignet ist das in EP 3,138,863 beschriebene N-Benzyl-1 ,2-ethandiamin. Damit erhaltene Epoxidharz-Beschichtungen sind aber in Bezug auf Verarbeitbarkeit, Aushärtegeschwindigkeit und Oberflächenqualität noch verbesserungsfähig.

Das Dokument DE-A-2853752 offenbart Verfahrens zum Herstellung von Klebstoffen auf der Basis von unmodifizierten, niedermolekularen, mindestens zwei Epoxidgruppen pro Molekül enthaltenden Epoxidharzen unter Verwendung von N,N'-Dibenzylethylendiamin als Polyadditionskomponente.

### Darstellung der Erfindung

Aufgabe der vorliegenden Erfindung ist es, einen Härter für Epoxidharze zur Verfügung zu stellen, welcher die Nachteile des Standes der Technik überwindet und sich insbesondere für ästhetisch anspruchsvolle Beschichtungsanswendungen eignet.

Diese Aufgabe wird mit dem im Anspruch 1 beschriebenen Härter enthaltend eine bestimmte Kombination aus N-Benzyl-1 ,2-ethandiamin und N,N'-Dibenzyl-1 ,2-ethandiamin gelöst. Der erfindungsgemässe Härter ist niedrigviskos und verdünnt eine Epoxidharz-Zusammensetzung besonders gut, wodurch diese emissionsarm formuliert werden kann und trotzdem als Beschichtung gut verarbeitbar ist. Weiterhin ermöglicht der erfindungsgemässe Härter eine überraschend schnelle Aushärtung bei feuchtkalten Bedingungen, wie insbesondere 8°C/80% relative Feuchtigkeit. Die Beschichtung ist dadurch schon nach kurzer Zeit, typischerweise innerhalb der ersten 24 Stunden nach der Applikation, begehbar und kann weiter bearbeitet, beispielsweise überbeschichtet oder versiegelt, werden. Und schliesslich erreicht der erfindungsgemässe Härter besonders gute Ergebnisse bei der Oberflächenqualität der ausgehärteten Beschichtung.

Im Vergleich zur Verwendung von N-Benzyl-1,2-ethandiamin alleine ermöglicht die Mitverwendung von N,N'-Dibenzyl-1,2-ethandiamin im beanspruchten Gewichtsverhältnis eine schnellere Aushärtung und eine höhere Oberflächenqualität bei feuchtkalten Bedingungen sowie eine bessere Verarbeitbarkeit, ohne dass dabei die Endhärte oder die Aushärtung im Normklima (23°C/50% relative Feuchtigkeit) deutlich beeinträchtigt werden. Dies ist besonders überraschend, würde man doch aufgrund der niedrigeren Funktionalität von N,N'-Dibenzyl-1,2-ethandiamin eine deutlich verlangsamte Aushärtung und eine geringere Endhärte erwarten.

Der erfindungsgemässe Härter ist einfach herstellbar, insbesondere über die reduktive Alkylierung von 1,2-Ethandiamin mit Benzaldehyd und Wasserstoff. Er verdünnt Epoxidharze besonders gut und ermöglicht emissionsarme Epoxidharz-Produkte mit guter Verarbeitbarkeit und langer Topfzeit, welche zuverlässig und überraschend schnell aushärten und bald begehbar sind, insbesondere auch bei feuchtkalten Bedingungen. Dabei entstehen insbesondere mechanisch hochwertige Beschichtungen mit hoher Oberflächenqualität und geringer Neigung zum Vergilben. Solche Epoxidharz-Produkte sind besonders geeignet als Beschichtung für Böden.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Härter für Epoxidharze enthaltend N-Benzyl-1,2-ethandiamin und N,N'-Dibenzyl-1,2-ethandiamin in einem Gewichtsverhältnis im Bereich von 65/35 bis 95/5, der eine Reaktionsmischung erhalten aus der reduktiven Alkylierung von 1,2-Ethandiamin mit Benzaldehyd und Wasserstoff, enthaltend N-Benzyl-1,2-ethandiamin und N,N'-Dibenzyl-1,2-ethandiamin in einem Gewichtsverhältnis im Bereich von 65/35 bis 95/5, enthält. Gegenstand der Erfindung sind auch die in den vorliegenden Ansprüchen beschriebenen Zusammensetzungen und Verfahren.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "Aminwasserstoff" werden die Wasserstoffatome von primären und sekundären Aminogruppen bezeichnet.

Als "Aminwasserstoff-Equivalentgewicht" wird die Masse eines Amins oder einer Amin-haltigen Zusammensetzung, die ein Molequivalent Aminwasserstoff enthält, bezeichnet.

Mit "Poly" beginnende Substanznamen wie Polyamin oder Polyepoxid bezeichnen Substanzen, die formal zwei oder mehr der in ihrem Namen vorkommenden funktionellen Gruppen pro Molekül enthalten.

Als "Verdünner" wird eine in einem Epoxidharz lösliche und dessen Viskosität senkende Substanz bezeichnet, welche bei der Aushärtung chemisch nicht in das Epoxidharz-Polymer eingebunden wird.

Als "Molekulargewicht" wird die molare Masse (in Gramm pro Mol) eines Moleküls bezeichnet. Als "mittleres Molekulargewicht" wird das Zahlenmittel Mₙ einer polydispersen Mischung von oligomeren oder polymeren Molekülen bezeichnet, welches üblicherweise mittels Gelpermeationschromatographie (GPC) gegen Polystyrol als Standard bestimmt wird.

Als "Topfzeit" wird die Verarbeitbarkeitsdauer einer Epoxidharz-Zusammensetzung bezeichnet, d.h. die maximal mögliche Zeitspanne zwischen dem Mischen der Komponenten und der Applikation der vermischten Zusammensetzung, in der diese in einem ausreichend fliessfähigen Zustand ist und die Substratoberflächen benetzen kann.

Als "Offenzeit" eines Klebstoffs wird die für eine kraftschlüssige Verbindung maximal mögliche Zeitspanne zwischen der Applikation des Klebstoffs und dem Fügen der zu verklebenden Teile bezeichnet.

Als "Raumtemperatur" wird eine Temperatur von 23°C bezeichnet.

Bevorzugt ist der Härter nicht wasserbasiert. Insbesondere enthält er weniger als 10 Gewichts-%, bevorzugt weniger als 5 Gewichts-%, besonders bevorzugt weniger als 2.5 Gewichts-%, Wasser. Ein solcher Härter ist besonders geeignet für die Aushärtung von nicht emulgierten Epoxidharzen und ermöglicht sehr hydrophobe und beständige Materialien.

Bevorzugt ist der Härter weitgehend frei von N,N-Dibenzyl-1,2-ethandiamin, also dem Diamin mit einer tertiären und einer primären Aminogruppe. Bevorzugt enthält der Härter weniger als 2 Gewichts-%, besonders bevorzugt wengiger als 1 Gewichts-%, insbesondere weniger als 0.5 Gewichts-%, N,N-Dibenzyl-1,2-ethandiamin bezogen auf die gesamte Menge an N-Benzyl-1,2-ethandiamin und N,N'-Dibenzyl-1,2-ethandiamin.

Bevorzugt liegt das Gewichtsverhältnis zwischen N-Benzyl-1,2-ethandiamin und N,N'-Dibenzyl-1,2-ethandiamin im Bereich von 70/30 bis 90/10, insbesondere 80/20 bis 90/10. Ein solcher Härter ermöglicht eine besonders attraktive Kombination aus guter Verdünnung, schneller Aushärtung und hoher Oberflächenqualität.

N-Benzyl-1,2-ethandiamin und N,N'-Dibenzyl-1,2-ethandiamin werden bevorzugt hergestellt über die reduktive Alkylierung von 1,2-Ethandiamin mit Benzaldehyd und Wasserstoff.

Die reduktive Alkylierung wird bevorzugt in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.

Die reduktive Alkylierung wird bevorzugt in einer Druckapparatur bei einem Wasserstoff-Druck von 5 bis 150 bar, insbesondere 10 bis 100 bar, durchgeführt. Dies kann in einem Batch-Prozess oder bevorzugt in einem kontinuierlichen Prozess erfolgen.

Die reduktive Alkylierung wird bevorzugt bei einer Temperatur im Bereich von 40 bis 120°C, insbesondere 60 bis 100°C, durchgeführt.

N-Benzyl-1,2-ethandiamin und N,N'-Dibenzyl-1,2-ethandiamin können einzeln hergestellt und im für den erfindungsgemässen Härter passenden Gewichtsverhältnis abgemischt werden.

Bevorzugt werden N-Benzyl-1,2-ethandiamin und N,N'-Dibenzyl-1,2-ethandiamin zusammen hergestellt, indem die reduktive Alkylierung in einem bestimmten Molverhältnis zwischen 1,2-Ethandiamin und Benzaldehyd durchgeführt wird, das zu einer Reaktionsmischung enthaltend beide Amine führt.

Besonders bevorzugt wird das Molverhältnis zwischen 1,2-Ethandiamin und Benzaldehyd so gewählt, dass das Gewichtsverhältnis zwischen N-Benzyl-1,2-ethandiamin und N,N'-Dibenzyl-1,2-ethandiamin in der erhaltenen Reaktionsmischung dem des erfindungsgemässen Härters entspricht.

Das Molverhältnis von 1,2-Ethandiamin zu Benzaldehyd liegt bevorzugt im Bereich von 5/1 bis 1.5/1, besonders bevorzugt im Bereich von 4/1 bis 2/1. Mit einer solchen Reaktionsmischung kann der erfindungsgemässe Härter direkt hergestellt werden, ohne dass eine Zugabe von separat hergestelltem N-Benzyl-1,2-ethandiamin oder N,N'-Dibenzyl-1,2-ethandiamin notwendig ist.

Bevorzugt enthält der erfindungsgemässe Härter somit eine Reaktionsmischung erhalten aus der reduktiven Alkylierung von 1,2-Ethandiamin mit Benzaldehyd und Wasserstoff, enthaltend N-Benzyl-1,2-ethandiamin und N,N'-Dibenzyl-1,2-ethandiamin in einem Gewichtsverhältnis im Bereich von 65/35 bis 95/5, bevorzugt 70/30 bis 90/10, insbesondere 80/20 bis 90/10.

Bevorzugt enthält der erfindungsgemässe Härter mindestens ein weiteres Amin.

Als weiteres Amin bevorzugt sind aliphatische, cycloaliphatische oder arylaliphatische Polyamine mit mindestens 2, insbesondere mindestens 3, Aminwasserstoffen, wie insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethyl-1,6-hexandiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, 1,3-Bis(aminomethyl)-cyclohexan, 1,4-Bis(aminomethyl)cyclohexan, Bis(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPDA), 2(4)-Methyl-1,3-diaminocyclohexan, 2,5(2,6)-Bis(aminomethyl)bicyclo[2.2.1]heptan (NBDA), 3(4),8(9)-Bis(aminomethyl)tricyclo[5.2.1.0^{2,6}]-decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)-benzol (MXDA), 1,4-Bis(aminomethyl)benzol, Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatride-can-1,13-diamin oder höhere Oligomere dieser Diamine, Bis(3-aminopropyl)polytetrahydrofuran oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine^{®} RFD-270 (von Huntsman), oder Polyoxyalkylendi- oder -triamine, insbesondere Jeffamine^{®} D-230, Jeffamine^{®} D-400, Jeffamine^{®} D-2000, Jeffamine^{®} EDR-104, Jeffamine^{®} EDR-148, Jeffamine^{®} EDR-176, Jeffamine^{®} T-403, Jeffamine^{®} T-3000, Jeffamine^{®} T-5000 (alle von Huntsman), oder entsprechende Amine von BASF oder Nitroil, 2-Aminoethylpiperazin, 3-Dimethylaminopropylamin (DMAPA), 3-(3-(Dimethylamino)propylamino)propylamin (DMAPAPA), Bis(6-aminohexyl)amin (BHMT), Diethylentriamin (DETA), Triethylentetramin (TETA), Tetraethylenpentamin (TEPA), Pentaethylenhexamin (PEHA) oder höhere Homologe linearer Polyethylenamine, Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-ami-nopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin, N,N'-Bis(3-amino-1-ethylpropyl)-2-methyl-1,5-pentandiamin, N-Benzyldiethylentriamin, N-Benzyltriethylentetramin, N,N'-Dibenzyltriethylentetra-min, N"-Benzyl-N,N'-bis(3-aminopropyl)ethylendiamin, N",N‴-Dibenzyl-N,N'-bis(3-aminopropyl)ethylendiamin, N-Benzyl-1,2-propandiamin, N,N'-Dibenzyl-1,2-propandiamin, N-Benzyl-1,3-bis(aminomethyl)benzol, N,N'-Dibenzyl-1,3-bis(aminomethyl)benzol, N-(2-Ethylhexyl)-1,3-bis(aminomethyl)benzol, N,N'-Bis(2-ethylhexyl)-1,3-bis(aminomethyl)benzol, styrolisiertes MXDA (erhältlich als Gaskamine^{®} 240 von Mitsubishi Gas Chemical), Addukte der obengenannten oder weiterer Polyamine mit Epoxiden oder Epoxidharzen, insbesondere Addukte mit Diepoxiden oder Monoepoxiden, oder Polyamidoamine, insbesondere Umsetzungsprodukte aus einer ein- oder mehrwertigen Carbonsäure oder deren Ester oder Anhydrid, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, oder Mannich-Basen, insbesondere Phenalkamine, also Umsetzungsprodukte von Phenolen, insbesondere Cardanol, mit Aldehyden, insbesondere Formaldehyd, und Polyaminen.

Bevorzugt enthält der Härter mindestens ein weiteres Amin ausgewählt aus der Gruppe bestehend aus TMD, 1,2-, 1,3- oder 1,4-Diaminocyclohexan, 1,3-Bis(aminomethyl)cyclohexan, 1,4-Bis(aminomethyl)cyclohexan, Bis(4-aminocyclohexyl)-methan, IPDA, 2(4)-Methyl-1,3-diaminocyclohexan, MXDA, Polyoxypropylendiaminen mit einem mittlerem Molekulargewicht Mₙ im Bereich von 200 bis 500 g/mol, DMAPAPA, BHMT, DETA, TETA, TEPA, PEHA, DPTA, N3-Amin, N4-Amin, Addukte dieser oder weiterer Polyamine mit Mono- oder Diepoxiden und Mannich-Basen.

Davon bevorzugt ist TMD, 1,3-Bis(aminomethyl)cyclohexan, IPDA, MXDA, DMAPAPA, BHMT, DETA, TETA, TEPA, PEHA, DPTA, N3-Amin, N4-Amin oder Addukte davon mit Mono- oder Diepoxiden, insbesondere Addukte davon mit Bisphenol-A oder Bisphenol-F Diglycidylether. Die Mitverwendung eines solchen Amins kann eine besonders hohe Härte oder eine besonders hohe Glasübergangstemperatur ermöglichen.

Davon bevorzugt ist weiterhin ein Addukt aus MPMD oder 1,2-Propandiamin mit Kresylglycidylether, insbesondere ortho-Kresylglycidylether. Die Adduktierung wird bevorzugt mit einem Überschuss an MPMD oder 1,2-Propandiamin gegenüber dem Kresylglycidylether durchgeführt und das nicht adduktierte Amin nach der Umsetzung mittels Destillation entfernt.

Bevorzugt kann der Härter eine Kombination aus zwei oder mehr der genannten weiteren Amine enthalten.

Besonders bevorzugt als weiteres Amin ist IPDA. Ein solcher Härter ist breit verfügbar, kostengünstig und ermöglicht Epoxidharz-Produkte mit besonders hoher Härte und Glasübergangstemperatur.

Besonders bevorzugt als weiteres Amin ist MXDA. Ein solcher Härter ist breit verfügbar, kostengünstig und ermöglicht Epoxidharz-Produkte mit besonders schneller Aushärtung und hoher Glasübergangstemperatur.

Besonders bevorzugt als weiteres Amin ist weiterhin TETA, TEPA, PEHA oder N4-Amin. Ein solcher Härter ist breit verfügbar, besonders kostengünstig und ermöglicht Epoxidharz-Produkte mit besonders schneller Aushärtung, hoher Härte und hoher Glasübergangstemperatur.

Besonders bevorzugt als weiteres Amin ist weiterhin DMAPAPA, insbesondere für die Verwendung in Epoxidharz-Klebstoffen. Damit werden besonders hohe Festigkeiten und Haftkräfte erhalten.

Bevorzugt kann der erfindungsgemässe Härter auch eine Kombination von zwei oder mehr solcher weiterer Amine enthalten.

Bevorzugt werden solche weiteren Amine in einer solchen Menge eingesetzt, dass pro mol Aminwasserstoff aus N-Benzyl-1,2-ethandiamin und N,N'-Dibenzyl-1,2-ethandiamin 0.1 bis 10, bevorzugt 0.2 bis 5, mol Aminwasserstoffe aus weiteren Aminen vorhanden sind.

Der Härter enthält gegebenenfalls mindestens einen Verdünner.

Geeignet sind insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykolmonomethylether, Diethylenglykolmonoethylether, Diethylenglykolmono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol, Cardanol (aus Cashewschalen-Öl, enthaltend als Hauptbestandteil 3-(8,11,14-Pentadecatrienyl)phenol), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide.

Bevorzugt sind Verdünner mit einem Siedepunkt von mehr als 200 °C. Bevorzugt ist der Verdünner ausgewählt aus der Gruppe bestehend aus Benzylalkohol, styrolisiertem Phenol, ethoxyliertem Phenol, phenolgruppenhaltigen aromatischen Kohlenwasserstoffharzen, insbesondere den Novares^{®}-Typen LS 500, LX 200, LA 300 oder LA 700 (von Rütgers), Diisopropylnaphthalin und Cardanol. Besonders bevorzugt ist Benzylalkohol.

Phenolgruppen-haltige Verdünner zeigen auch eine Wirkung als Beschleuniger.

Der Härter enthält gegebenenfalls mindestens einen Beschleuniger.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; Nitrate wie insbesondere Calciumnitrat; tertiäre Amine wie insbesondere 1,4-Diazabicyclo[2.2.2]octan, Benzyldimethylamin, α-Methylbenzyldimethylamin, Triethanolamin, Dimethylaminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo-[5.4.0]undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)-phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen.

Als Beschleuniger bevorzugt sind Säuren, Nitrate, tertiäre Amine oder Mannich-Basen.

Besonders bevorzugt ist Salicylsäure oder Calciumnitrat oder 2,4,6-Tris(dimethylaminomethyl)phenol oder eine Kombination davon.

Am meisten bevorzugt ist eine Kombination aus Calciumnitrat und 2,4,6-Tris(dimethylaminomethyl)phenol. Damit werden eine besonders schnelle Aushärtung, insbesondere auch bei kalten Temperaturen, und besonders hohe Härten erreicht.

Calciumnitrat wird insbesondere in Form einer wässrigen Lösung mit 20 bis 70 Gewichts-% Calciumnitrat eingesetzt.

Ein weiterer Gegenstand der vorliegenden Erfindung ist eine Epoxidharz-Zusammensetzung umfassend
- eine Harz-Komponente enthaltend mindestens ein Epoxidharz und
- eine Härter-Komponente umfassend den vorgänig beschriebenen Härter enthaltend N-Benzyl-1,2-ethandiamin und N,N'-Dibenzyl-1,2-ethandiamin in einem Gewichtsverhältnis im Bereich von 65/35 bis 95/5, bevorzugt 70/30 bis 90/10, insbesondere 80/20 bis 90/10.

Ein geeignetes Epoxidharz wird auf bekannte Art und Weise erhalten, insbesondere aus der Oxidation von Olefinen oder aus der Reaktion von Epichlorhydrin mit den Polyolen, Polyphenolen oder Aminen.

Geeignete Epoxidharze sind insbesondere aromatische Epoxidharze, insbesondere die Glycidylether von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)-methan, 2,2-Bis(4-hydroxy-3-methylphenyl)propan (Bisphenol-C), Bis(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis(3,5-dimethyl-4-hydroxyphenyl)-2-methylbutan, 1,1-Bis(4-hydroxyphenyl)cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)-ethan, Bis(4-hydroxyphenyl)ether oder Bis(4-hydroxyphenyl)sulfon;
- Novolaken, welche insbesondere Kondensationsprodukte von Phenol oder Kresolen mit Formaldehyd bzw. Paraformaldehyd oder Acetaldehyd oder Crotonaldehyd oder Isobutyraldehyd oder 2-Ethylhexanal oder Benzaldehyd oder Furfural sind;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂- bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Bevorzugt ist das Epoxidharz ein Flüssigharz oder eine Mischung enthaltend zwei oder mehr Epoxid-Flüssigharze.

Als "Epoxid-Flüssigharz" wird ein technisches Polyepoxid mit einer Glasübergangstemperatur unterhalb von 25°C bezeichnet.

Gegebenenfalls enthält die Harz-Komponente zusätzlich Anteile von Epoxid-Festharz.

Das Epoxidharz ist insbesondere ein Flüssigharz auf der Basis eines Bisphenols, insbesondere ein Bisphenol-A Diglycidylether und/oder Bisphenol-F-Diglycidylether, wie sie kommerziell beispielsweise von Olin, Huntsman oder Momentive erhältlich sind. Diese Flüssigharze weisen eine für Epoxidharze niedrige Viskosität auf und ermöglichen eine schnelle Aushärtung und hohe Härten. Sie können Anteile von Bisphenol A-Festharz oder Novolak-Glycidylethern enthalten.

Die Harz-Komponente kann einen Reaktivverdünner enthalten.

Als Reaktivverdünner bevorzugt sind Epoxidgruppen-haltige Reaktivverdünner, insbesondere Butandioldiglycidylether, Hexandioldiglycidylether, Trimethylolpropandi- oder triglycidlyether, Phenylglycidylether, Kresylglycidylether, Guaiacolglycidylether, 4-Methoxyphenylglycidylether, p-n-Butylphenylglycidylether, p-tert.Butylphenylglycidylether, 4-Nonylphenylglycidylether, 4-Dodecylphenylglycidylether, Cardanolglycidylether, Benzylglycidylether, Allylglycidylether, Butylglycidylether, Hexylglycidylether, 2-Ethylhexylglycidylether, oder Glycidylether von natürlichen Alkoholen wie insbesondere C₈- bis C₁₀- oder C₁₂- bis C₁₄- oder C₁₃-bis C₁₅-Alkylglycidylether.

Bevorzugt enthält die Epoxidharz-Zusammensetzung mindestens einen weiteren Bestandteil ausgewählt aus der Gruppe bestehend aus Verdünnern, Beschleunigern und Füllstoffen.

Als Beschleuniger geeignet sind die bereits genannten, insbesondere Salicylsäure, Calciumnitrat oder 2,4,6-Tris(dimethylaminomethyl)phenol oder eine Kombination davon. Am meisten bevorzugt ist eine Kombination aus Calciumnitrat und 2,4,6-Tris(dimethylaminomethyl)phenol. Damit werden eine besonders schnelle Aushärtung, insbesondere auch bei kalten Temperaturen, und besonders hohe Härten erreicht.

Als Verdünner geeignet sind die bereits genannten, insbesondere solche mit einem Siedepunkt von mehr als 200°C.

Bevorzugt ist der Verdünner ausgewählt aus der Gruppe bestehend aus Benzylalkohol, styrolisiertem Phenol, ethoxyliertem Phenol, phenolgruppenhaltigen aromatischen Kohlenwasserstoffharzen, insbesondere den Novares^{®}-Typen LS 500, LX 200, LA 300 oder LA 700 (von Rütgers), Diisopropylnaphthalin und Cardanol. Besonders bevorzugt ist Benzylalkohol.

Bevorzugt enthält die Epoxidharz-Zusammensetzung nur einen geringen Gehalt an Verdünnern. Bevorzugt enthält sie weniger als 25 Gewichts-%, besonders bevorzugt weniger als 15 Gewichts-%, insbesondere weniger als 10 Gewichts-%, Verdünner. Dies ermöglicht emissionsarme oder emissionsfreie Epoxidharz-Produkte.

Geeignete Füllstoffe sind insbesondere gemahlenes oder gefälltes Calciumcarbonat, welches gegebenenfalls mit Fettsäure, insbesondere Stearaten, beschichtet ist, Baryt (Schwerspat), Talk, Quarzmehl, Quarzsand, Siliciumcarbid, Eisenglimmer, Dolomit, Wollastonit, Kaolin, Mica (Kalium-Aluminium-Silikat), Molekularsieb, Aluminiumoxid, Aluminiumhydroxid, Magnesiumhydroxid, Kieselsäure, Zement, Gips, Flugasche, Russ, Graphit, Metall-Pulver wie Aluminium, Kupfer, Eisen, Zink, Silber oder Stahl, PVC-Pulver oder Hohlkugeln.

Bevorzugt ist Calciumcarbonat, Quarzmehl und Quarzsand.

Gegebenenfalls enthält die Epoxidharz-Zusammensetzung weitere Hilfs- und Zusatzstoffe, insbesondere die Folgenden:
- Reaktivverdünner, insbesondere die bereits vorgängig erwähnten, oder epoxidiertes Sojaöl oder Leinöl, Acetoacetatgruppen aufweisende Verbindungen, insbesondere acetoacetylierte Polyole, Butyrolakton, Carbonate, Aldehyde, Isocyanate oder Reaktivgruppen aufweisende Silikone;
- Lösemittel;
- weitere Amine, insbesondere Monoamine wie insbesondere Benzylamin oder Furfurylamin oder aromatische Polyamine wie insbesondere 4,4'-, 2,4' und/oder 2,2'-Diaminodiphenylmethan, 2,4- und/oder 2,6-Toluylendiamin, 3,5-Dimethyl-thio-2,4- und/oder -2,6-toluylendiamin, 3,5-Diethyl-2,4- und/oder -2,6-toluylendiamin;
- Mercaptogruppen aufweisende Verbindungen, insbesondere flüssige Mercaptan-terminierte Polysulfidpolymere, Mercaptan-terminierte Polyoxyalkylenether, Mercaptan-terminierte Polyoxyalkylen-Derivate, Polyester von Thiocarbonsäuren, 2,4,6-Trimercapto-1,3,5-triazin, Triethylenglykoldimercaptan oder Ethandithiol;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere oder Sulfonamidmodifizierte Melamine;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid, Eisenoxide oder Chrom(lll)oxid;
- Rheologie-Modifizierer, insbesondere Verdicker oder Antiabsetzmittel;
- Haftverbesserer, insbesondere Organoalkoxysilane;
- flammhemmende Substanzen, insbesondere die bereits genannten Füllstoffe Aluminiumhydroxid oder Magnesiumhydroxid, Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat, Bisphenol-A-bis(diphenylphosphat), Tris(chloroethyl)phosphat, Tris(chloropropyl)-phosphat, Tris(dichloroisopropyl)phosphat, Tris[3-bromo-2,2-bis(bromomethyl)-propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine; oder
- Additive, insbesondere dispergiertes Paraffinwachs, Filmbildehilfsmittel, Netzmittel, Verlaufmittel, Entschäumer, Entlüfter, Stabilisatoren gegen Oxidation, Wärme, Licht oder UV-Strahlung oder Biozide.

Bevorzugt enthält die Epoxidharz-Zusammensetzung weitere Hilfs- und Zusatzstoffe, insbesondere Pigmente, Netzmittel, Verlaufsmittel und/oder Entschäumer.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.

Die in der Epoxidharz-Zusammensetzung vorhandenen primären und sekundären Aminogruppen und gegebenenfalls vorhandene weitere gegenüber Epoxidgruppen reaktive Gruppen reagieren mit den Epoxidgruppen unter deren Ringöffnung (Additionsreaktion). Als Ergebnis hauptsächlich dieser Reaktion polymerisiert die Zusammensetzung und härtet dadurch aus.

Die Harz- und die Härter-Komponente der Epoxidharz-Zusammensetzung werden in voneinander getrennten Gebinden gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ebenfalls möglich ist, dass weitere Bestandteile als eigene, weitere Komponente vorhanden sind.

Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Komponenten kurz vor oder während der Applikation miteinander vermischt. Das Mischungsverhältnis zwischen der Harz- und der Härter-Komponente wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.

Die Vermischung der Komponenten erfolgt mittels eines geeigneten Verfahrens; sie kann kontinuierlich oder batchweise erfolgen. Falls das Vermischen nicht unmittelbar vor der Applikation erfolgt, muss darauf geachtet werden, dass zwischen dem Vermischen der Komponenten und der Applikation nicht zu viel Zeit vergeht und die Applikation innerhalb der Topfzeit erfolgt. Die Vermischung erfolgt insbesondere bei Umgebungstemperatur, welche typischerweise im Bereich von etwa 5 bis 40°C, bevorzugt bei etwa 10 bis 35°C, liegt.

Mit dem Vermischen der beiden Komponenten beginnt die Aushärtung durch chemische Reaktion, wie vorgängig beschrieben. Die Aushärtung erfolgt typischerweise bei einer Temperatur im Bereich von 0 bis 150°C. Bevorzugt erfolgt sie bei Umgebungstemperatur und erstreckt sich typischerweise über einige Tage bis Wochen. Die Dauer hängt unter anderem von der Temperatur, der Reaktivität der Bestandteile und deren Stöchiometrie sowie der Gegenwart von Beschleunigern ab.

Die Applikation der Epoxidharz-Zusammensetzung erfolgt auf mindestens ein Substrat, wobei die Folgenden besonders geeignet sind:
- Glas, Glaskeramik, Beton, Mörtel, Zementestrich, Faserzement, Backstein, Ziegel, Gips oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Eisen, Stahl, Kupfer, weitere Buntmetalle, inklusive oberflächenveredelte Metalle oder Legierungen wie verzinkte oder verchromte Metalle
- Asphalt oder Bitumen;
- Leder, Textilien, Papier, Holz, mit Harzen, beispielsweise Phenol-, Melamin-oder Epoxidharzen, gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Beschichtungen, Farben oder Lacke, insbesondere beschichtete Böden, welche mit einer weiteren Bodenbelagsschicht überschichtet werden.

Die Substrate können bei Bedarf vor dem Applizieren vorbehandelt werden, insbesondere durch physikalische und/oder chemische Reinigungsverfahren oder das Aufbringen eines Aktivators oder eines Primers.

Aus der Aushärtung der beschriebenen Epoxidharz-Zusammensetzung wird eine ausgehärtete Zusammensetzung erhalten.

Die beschriebene Epoxidharz-Zusammensetzung wird bevorzugt verwendet als Beschichtung, Primer, Klebstoff, Dichtstoff, Vergussmasse, Giessharz oder als Matrix für Faserverbundwerkstoffe (Composites) wie insbesondere CFK oder GFK. Unter den Begriff der Beschichtung fallen auch Grundierungen, Anstriche, Lacke und Versiegelungen.

Besonders bevorzugt wird die beschriebene Epoxidharz-Zusammensetzung als Beschichtung verwendet. Als Beschichtung werden dabei flächig aufgebrachte Beläge aller Art verstanden, insbesondere Bodenbeläge, Anstriche, Lacke, Versiegelungen, Grundierungen, Primer oder Schutzbeschichtungen, insbesondere auch solche für schweren Korrosionsschutz.

Besonders geeignet ist die Epoxidharz-Zusammensetzung als Bodenbelag oder Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden, oder als Voranstrich, Haftanstrich, Korrosionsschutz-Primer oder zur Hydrophobierung von Oberflächen.

Besonders vorteilhaft wird die beschriebene Epoxidharz-Zusammensetzung in emissionsarmen Beschichtungen mit Öko-Gütesiegeln, beispielsweise nach Emicode (EC1 Plus), AgBB, DIBt, Der Blaue Engel, AFSSET, RTS (M1) und US Green Building Council (LEED), verwendet.

Für die Verwendung als Beschichtung weist die Epoxidharz-Zusammensetzung vorteilhaft eine flüssige Konsistenz mit niedriger Viskosität und guten Verlaufseigenschaften auf. Die vermischte Zusammensetzung wird innerhalb der Topfzeit typischerweise flächig als dünner Film mit einer Schichtdicke von etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann auch mit einem Pinsel oder Roller oder als Spritzapplikation erfolgen, beispielsweise als Korrosionsschutzbeschichtung auf Stahl. Bei der Aushärtung entstehen typischerweise weitgehend homogene, glänzende und nichtklebrige Filme von hoher Härte, welche eine gute Haftung zu verschiedensten Substraten aufweisen.

Ein weiterer Gegenstand der Erfindung ist ein Verfahren zum Beschichten umfassend die Schritte
(i) Vermischen der Komponenten der beschriebenen Epoxidharz-Zusammensetzung,
(ii) Applizieren der vermischten Zusammensetzung auf ein Substrat innerhalb der Topfzeit,
gefolgt von der Aushärtung der vermischten Zusammensetzung.

Auf die vollständig oder teilweise ausgehärtete Zusammensetzung kann eine weitere Beschichtung appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Weiterhin besonders bevorzugt wird die beschriebene Epoxidharz-Zusammensetzung als Klebstoff verwendet. Typischerweise weist die Epoxidharz-Zusammensetzung bei der Verwendung als Klebstoff nach dem Vermischen der Komponenten eine pastöse Konsistenz mit strukturviskosen Eigenschaften auf. Bei der Applikation wird der vermischte Klebstoff innerhalb der Topfzeit auf mindestens eines der zu verklebenden Substrate aufgetragen und die beiden Substrate innerhalb der Offenzeit des Klebstoffs zu einer Verklebung gefügt.

Der vermischte Klebstoff wird insbesondere mittels Pinsel, Rolle, Spatel, Rakel, Kelle, oder aus einer Tube, Kartusche oder Dosiervorrichtung appliziert bzw. aufgetragen.

Der Klebstoff ist besonders geeignet für Verwendungen in der Bauindustrie, insbesondere für die Armierung von Bauwerken mittels Stahllamellen oder Lamellen aus Kohlefaser-verstärkten Composite-Kunststoffen (CFK), für Konstruktionen, welche geklebte Beton-Fertigteile enthalten, insbesondere Brücken oder Betontürme für beispielsweise Windkraftanlagen, Schächte, Rohrleitungen oder Tunnels, oder für Konstruktionen, welche geklebte Natursteine, keramische Elemente oder Teile aus Faserzement, Stahl, Gusseisen, Aluminium, Holz oder Polyester enthalten, für die Verankerung von Dübeln oder Stahlstäben in Bohrlöchern, für die Fixierung von beispielsweise Geländern, Brüstungen oder Türrahmen, für Reparaturen wie insbesondere die Verfüllung von Kanten, Löchern oder Fugen bei der Betoninstandsetzung, oder für das Aufkleben von Folien aus Polyvinylchlorid (PVC), flexibilisiertem Polyolefin (Combiflex^{®}) oder haftungsmodifiziertem chlorsulfoniertem Polyethylen (Hypalon^{®}) auf Beton oder Stahl.

Weitere Einsatzgebiete betreffen das strukturelle Kleben in der Bau- oder Fertigungsindustrie, insbesondere als Klebemörtel, Montageklebstoff, Armierungsklebstoff wie insbesondere für das Verkleben von Lamellen aus CFK oder Stahl auf Beton, Mauerwerk oder Holz, als Element-Klebstoff für beispielsweise Brückenelemente, Sandwichelementklebstoff, Fassadenelementklebstoff, Verstärkungsklebstoff, Karrosserieklebstoff oder Halbschalenklebstoff für Rotorblätter von Windturbinen.

Ebenfalls geeignet ist ein solcher Epoxidharz-Klebstoff für das Verfüllen von Hohlräumen wie Rissen, Spalten oder Bohrlöchern, wobei der Klebstoff in den Hohlraum gefüllt oder injiziert wird und nach der Aushärtung diesen ausfüllt und die Flanken des Hohlraums kraftschlüssig miteinander verbindet bzw. verklebt.

Ein weiterer Gegenstand der Erfindung ist ein Verfahren zum Verkleben umfassend die Schritte
(i) Vermischen der Komponenten der beschriebenen Epoxidharz-Zusammensetzung,
(ii) Applizieren der vermischten Zusammensetzung innerhalb der Topfzeit,
   - entweder auf mindestens eines der zu verklebenden Substrate und Fügen der Substrate zu einer Verklebung innerhalb der Offenzeit,
   - oder in einen Hohlraum oder Spalt zwischen mehreren Substraten und gegebenenfalls Einfügen eines Ankers in den Hohlraum oder Spalt innerhalb der Offenzeit,
   gefolgt von der Aushärtung der vermischten Zusammensetzung.

Als "Anker" wird dabei insbesondere ein Armierungseisen, ein Gewindestab oder ein Bolzen bezeichnet. Ein solcher wird insbesondere so in einer Mauer, Wand, Decke oder in einem Fundament eingeklebt bzw. verankert, dass ein Teil davon kraftschlüssig verklebt ist und ein Teil davon vorsteht und konstruktiv belastet werden kann.

Verklebt werden können gleichartige oder verschiedene Substrate.

Aus der Applikation und Aushärtung der beschriebenen Epoxidharz-Zusammensetzung bzw. aus dem Verfahren zum Beschichten oder dem Verfahren zum Verkleben wird ein Artikel erhalten, welcher mit der Zusammensetzung beschichtet oder verklebt ist. Dieser Artikel kann ein Bauwerk oder ein Teil davon sein, insbesondere ein Bauwerk des Hoch- oder Tiefbaus, ein Büro, eine Industriehalle, eine Turnhalle, ein Kühlraum, ein Silo, eine Brücke, ein Dach, ein Treppenhaus, ein Balkon, eine Terrasse oder ein Parkdeck, oder er kann ein industrielles Gut oder ein Konsumgut sein, insbesondere ein Pier, eine Offshore-Plattform, ein Schleusentor, ein Kran, eine Spundwand, eine Rohrleitung oder ein Rotorblatt einer Windkraftanlage, oder ein Transportmittel wie insbesondere ein Automobil, ein Lastkraftwagen, ein Schienenfahrzeug, ein Schiff, ein Flugzeug oder ein Helikopter, oder ein Anbauteil davon.

Ein weiterer Gegenstand der Erfindung ist somit ein Artikel, erhalten aus der beschriebenen Verwendung oder dem beschriebenen Verfahren zum Beschichten oder dem beschriebenen Verfahren zum Verkleben.

Die beschriebene Epoxidharz-Zusammensetzung zeichnet sich durch vorteilhafte Eigenschaften aus. Sie ist auch mit wenig oder ganz ohne Verdünner niedrigviskos und gut verarbeitbar, verfügt über eine lange Topfzeit, härtet zuverlässig und schnell aus und ist bald begehbar, insbesondere auch bei feuchtkalten Bedingungen. Dabei entstehen insbesondere mechanisch hochwertige Beschichtungen mit hoher Oberflächenqualität und geringer Neigung zum Vergilben. Solche Epoxidharz-Produkte sind besonders geeignet als Beschichtungen, insbesondere für Böden.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" ("NK") wird eine Temperatur von 23±1°C und eine relative Luftfeuchtigkeit von 50±5% bezeichnet.

### Beschreibung der Messmethoden:

Die **Viskosität** wurde auf einem thermostatisierten Kegel-Platten-Viskosimeter Rheotec RC30 (Kegeldurchmesser 50 mm, Kegelwinkel 1°, Kegelspitze-PlattenAbstand 0.05 mm, Scherrate 10 s⁻¹) gemessen.

Die **Aminzahl** wurde durch Titration bestimmt (mit 0.1_{N} HClO₄ in Essigsäure gegen Kristallviolett).

### Verwendete Substanzen und Abkürzungen:

| | |
|---|---|
| Sikafloor^{®}-264N (A) | Sikafloor^{®}-264N Komponente A (RAL 5005), gefüllte pigmentierte Harzkomponente einer Epoxidharz-Bodenbeschichtung, EEW 450 g/Eq (von Sika) |
| B-EDA | N-Benzyl-1,2-ethandiamin, hergestellt wie nachfolgend beschrieben, AHEW 50 g/Eq |
| DB-EDA | N,N'-Dibenzyl-1,2-ethandiamin, hergestellt wie nachfolgend beschrieben, AHEW 120 g/Eq |
| B-EDA-mix | Reaktionsmischung enthaltend N-Benzyl-1,2-ethandiamin und N,N'-Dibenzyl-1,2-ethandiamin in einem Gewichtsverhältnis von 85/15, hergestellt wie nachfolgend beschrieben, AHEW 55 g/Eq |
| TEPA | Tetraethylenpentamin, AHEW 30 g/Eq (technisch, von Huntsman) |
| IPDA | 3-Aminomethyl-3,5,5-trimethylcyclohexylamin, AHEW42.6 g/Eq (Vestamin^{®} IPD, von Evonik) |
| Addukt-1 | Addukt aus 1,2-Propylendiamin und technischem o-Kresylglycidylether, hergestellt wie nachfolgend beschrieben, AHEW 90 g/Eq |
| Ca-Nitrat-Lösung | 50 Gewichts-% Calciumnitrat-Tetrahydrat in Wasser |
| Ancamine^{®} K54 | 2,4,6-Tris(dimethylaminomethyl)phenol (von Air Products) |

### Reaktionsmischung enthaltend N-Benzyl-1,2-ethandiamin (B-EDA-mix):

In einem Rundkolben wurden 180.3 g (3 mol) 1,2-Ethylendiamin unter Stickstoffatmosphäre bei Raumtemperatur vorgelegt. Unter gutem Rühren wurde langsam eine Lösung aus 106.0 g (1 mol) Benzaldehyd in 1200 ml Isopropanol dazugetropft und 2 Stunden nachgerührt. Die Reaktionsmischung wurde anschliessend bei einem Wasserstoff-Druck von 80 bar, einer Temperatur von 80°C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65°C eingeengt, wobei unreagiertes 1,2-Ethylendiamin, Wasser und Isopropanol entfernt wurden. Die so erhaltene Reaktionsmischung war eine klare, leicht gelbliche Flüssigkeit mit einer Aminzahl von 678 mg KOH/g und einem mittels GC bestimmten Gehalt an N-Benzyl-1,2-ethandiamin von ca. 81 Gewichts-% (Retentionszeit 8.47 - 8.57 min) und N,N'-Dibenzyl-1,2-ethandiamin von ca. 14 Gewichts-% (Retentionszeit 14.27 min). Dies entspricht einem Gewichtsverhältnis zwischen N-Benzyl-1,2-ethandiamin und N,N'-Dibenzyl-1,2-ethandiamin von 85/15.

### N-Benzyl-1,2-ethandiamin (B-EDA):

50 g der Reaktionsmischung enthaltend N-Benzyl-1,2-ethandiamin (B-EDA-mix), hergestellt wie vorgängig beschrieben, wurden bei 80°C unter Vakuum destilliert, wobei 31.3 g Destillat bei einer Dampftemperatur von 60 bis 65°C und 0.06 mbar aufgefangen wurden. Erhalten wurde eine farblose Flüssigkeit mit einer Viskosität von 8 mPa·s bei 20°C, einer Aminzahl von 750 mg KOH/g und einer mittels GC bestimmten Reinheit von > 97% (Retentionszeit 8.47 - 8.57 min).

### N,N'-Dibenzyl-1,2-ethandiamin (DB-EDA):

In einem Rundkolben wurden 30.1 g (0.5 mol) 1,2-Ethylendiamin unter Stickstoffatmosphäre bei Raumtemperatur vorgelegt. Unter gutem Rühren wurde langsam eine Lösung aus 111.5 g (1.1 mol) Benzaldehyd in 1'000 ml Isopropanol dazugetropft und 2 Stunden nachgerührt. Die Reaktionsmischung wurde anschliessend bei einem Wasserstoff-Druck von 80 bar, einer Temperatur von 80°C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65°C eingeengt, wobei unreagierter Benzaldehyd, Wasser und Isopropanol entfernt wurden. Die so erhaltene Reaktionsmischung war eine klare, leicht gelbliche Flüssigkeit.

100 g dieser Reaktionsmischung wurden bei 165°C unter Vakuum destilliert, wobei 90 g Destillat bei einer Dampftemperatur von 135°C und 0.03 mbar aufgefangen wurden. Erhalten wurde eine farblose Flüssigkeit mit einer Viskosität von 31 mPa·s bei 20°C, einer Aminzahl von 460 mg KOH/g und einer mittels GC bestimmten Reinheit von 96 % (Retentionszeit 14.27 min).

### Addukt-1:

Es wurden 4.15 kg 1,2-Propylendiamin unter Stickstoffatmosphäre vorgelegt, auf 70°C aufgewärmt und dann unter gutem Rühren langsam mit 2.93 kg Araldite^{®} DY-K (o-Kresylglycidylether technisch, von Huntsman) versetzt, wobei die Temperatur der Reaktionsmischung 70 bis 80°C betrug. Nach 1 Stunde bei 80°C wurde die Reaktionsmischung abgekühlt und die flüchtigen Bestandteile destillativ mittels Dünnschichtverdampfer (0.5-1 mbar, Manteltemperatur 115°C) entfernt.

### Herstellung von Epoxidharz-Zusammensetzungen:

### Beispiele 1 bis 6:

Für jedes Beispiel wurden die in der Tabelle 1 angegebenen Inhaltsstoffe der Härter-Komponente in den angegebenen Mengen (in Gewichtsteilen) mittels eines Zentrifugalmischers (SpeedMixer^{™} DAC 150, FlackTek Inc.) vermischt und unter Ausschluss von Feuchtigkeit aufbewahrt.

Als Harz-Komponente wurde Sikafloor^{®}-264N Komp. A (blau) (von Sika) in der in der Tabelle 1 angegebenen Menge (in Gewichtsteilen) eingesetzt. Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C gemessen ("**Viskosität (10')**").

Für die Bestimmung der **Shore D-Härte** nach DIN 53505 wurden je zwei zylindrische Prüfkörper (Durchmesser 20 mm, Dicke 5 mm) hergestellt. Einer wurde im Normklima gelagert und die Härte nach 1 Tag und nach 2 Tagen gemessen (1d NK) bzw. (2d NK), der andere wurde bei 8°C und 80% relativer Feuchtigkeit gelagert und die Härte nach 1 Tag und nach 2 Tagen im kalten Zustand gemessen (1d 8°/80%) bzw. (2d 8°/80%).

Ein erster Film wurde in einer Schichtdicke von 500 µm auf eine Glasplatte aufgezogen und dieser im Normklima gelagert bzw. ausgehärtet. An diesem Film wurde die **Königshärte** (Pendelhärte nach König, gemessen nach DIN EN ISO 1522) nach 1 Tag ("Königshärte (1d NK)"), nach 2 Tagen ("Königshärte (2d NK)"), nach 4 Tagen ("Königshärte (4d NK)"), nach 7 Tagen ("Königshärte (7d NK)") und nach 14 Tagen ("Königshärte (14d NK)") bestimmt. Nach 14 Tagen wurde der Aspekt des Films beurteilt (in der Tabelle mit "**Aspekt (NK)**" bezeichnet). Als "schön" wurde dabei ein Film bezeichnet, welcher eine glänzende und nichtklebrige Oberfläche ohne Struktur aufwies. Als "Struktur" wird dabei jegliche Art von Zeichnung oder Muster auf der Oberfläche bezeichnet. Als "matt" wurde ein Film mit einer nichtklebrigen Oberfläche ohne Struktur mit reduziertem Glanz bezeichnet.

Ein zweiter Film wurde in einer Schichtdicke von 500 µm auf eine Glasplatte aufgezogen und dieser unmittelbar nach dem Applizieren während 7 Tagen bei 8°C und 80% relativer Feuchtigkeit und anschliessend während 2 Wochen im Normklima gelagert, beziehungsweise ausgehärtet. 24 Stunden nach der Applikation wurde ein Flaschendeckel aus Polypropylen auf den Film aufgesetzt, unter welchem ein feuchter Schwamm platziert war. Nach weiteren 24 Stunden wurden der Schwamm und der Deckel entfernt und an einer neuen Stelle des Films platziert, wo sie nach 24 Stunden wieder entfernt und neu platziert wurden, insgesamt 4 mal. Anschliessend wurde der Aspekt dieses Films beurteilt (in den Tabellen mit "**Aspekt (8°/80%)**" bezeichnet), auf die gleiche Weise wie für den Aspekt (NK) beschrieben. Dabei wurde jeweils auch die Anzahl und Art der sichtbaren Marken angegeben, die im Film durch den feuchten Schwamm oder den aufgesetzten Deckel entstanden waren. Als "Blushing" wurde die Anzahl weiss verfärbte Flecken angegeben. Als "Ring" wurde die Stärke eines allfälligen ringförmigen Abdrucks durch Einsinken des ersten, 24h nach Applikation aufgesetzten Deckels angegeben. Ein solcher ringförmiger Abdruck zeigt an, dass die Beschichtung noch nicht begehbar ist. An den so ausgehärteten Filmen wurde wiederum die Königshärte bestimmt, jeweils nach 7 Tagen bei 8°C und 80% relativer Feuchtigkeit ("Königsh. (7d 8°/80%)"), dann nach weiteren 2 Tagen im NK ("Königsh. (+2d NK)") bzw. 7 Tagen im NK ("Königsh. (+7d NK)") bzw. 14d im NK ("Königsh. (+14d NK)").

Als Mass für die Vergilbung wurde weiterhin die Farbveränderung nach Belastung in einem Bewitterungstester bestimmt. Dazu wurde ein weiterer Film in einer Schichtdicke von 500 µm auf eine Glasplatte aufgezogen und dieser im Normklima während 2 Wochen gelagert bzw. ausgehärtet und anschliessend in einem Bewitterungstester des Typs Q-Sun Xenon Xe-1 mit optischem Filter Q-SUN Daylight-Q und einer Xenon Lampe mit einer Lichtstärke von 0.51 W/m² bei 340 nm bei einer Temperatur von 65°C während 72 Stunden belastet (**Q-Sun (72h)**). Anschliessend wurde der Farbunterschied ΔE des so belasteten Films im Vergleich zum entsprechenden nicht belasteten Film mittels einem Colorimeter NH310 von Shenzen 3NH Technology Co. LTD, ausgerüstet mit Silicon Photoelectric Diode Detector, Light Source A, Color Space Measurement Interface CIE L*a*b*C*H*, bestimmt. ΔE-Werte bis 5 stehen für eine leichte Vergilbung.

Die Resultate sind in der Tabelle 1 angegeben.

Die mit "(Ref.)" bezeichneten Beispiele sind Vergleichsbeispiele.

**Tabelle 1: Zusammensetzung und Eigenschaften der Beispiele 1 bis 6.**

| **Beispiel** | | **1 (Ref.)** | **2** | **3** | **4 (Ref.)** | **5 (Ref.)** | **6** |
|---|---|---|---|---|---|---|---|
| **Harz-Komp.:** | | | | | | | |
| Sikafloor^{®}-264N (A) | | 450.0 | 450.0 | 450.0 | 450.0 | 450.0 | 450.0 |
| **Härter-Komp.:** | | | | | | | |
| **B-EDA** | | 42.6 | - | 39.7 | 33.3 | 35.1 | - |
| **DB-EDA** | | - | - | 7.0 | 22.2 | - | - |
| **B-EDA-mix** | | - | 46.7 | - | - | - | 38.5 |
| TEPA | | 4.5 | 4.5 | 4.5 | 4.5 | 4.5 | 4.5 |
| Addukt-1 | | - | - | - | - | 13.5 | 13.5 |
| Benzylalkohol | | 10.0 | 10.0 | 10.0 | 10.0 | 10.0 | 10.0 |
| Ca-Nitrat-Lösung | | 2.0 | 2.0 | 2.0 | 2.0 | 2.0 | 2.0 |
| Ancamine^{®} K54 | | 2.0 | 2.0 | 2.0 | 2.0 | 2.0 | 2.0 |
| Gewichtsverhältnis¹ | | 100/0 | 85/15 | 85/15 | 60/40 | 100/0 | 85/15 |
| Viskosität (10') [Pa·s] | | 1.9 | 1.8 | 1.8 | 1.8 | 2.5 | 2.3 |
| Shore D | (1d NK) | 76 | 72 | 72 | 67 | 66 | 63 |
| | (2d NK) | 80 | 76 | 76 | 71 | 71 | 73 |
| Shore D | (1d 8°/80%) | 42 | 43 | 49 | 17 | 38 | 35 |
| | (2d 8°/80%) | 72 | 73 | 75 | 55 | 65 | 64 |
| Königshärte [s] | (1d NK) | 63 | 60 | 62 | 43 | 52 | 45 |
| | (2d NK) | 84 | 79 | 85 | 72 | 87 | 74 |
| | (4d NK) | 102 | 92 | 99 | 88 | 104 | 92 |
| | (7d NK) | 112 | 105 | 115 | 98 | 126 | 116 |
| | (14d NK) | 119 | 115 | 116 | 101 | 150 | 133 |
| Aspekt (NK) | | schön | schön | schön | schön | schön | schön |
| Q-Sun (72h) ΔE | | 3.7 | 4.3 | 4.9 | 3.4 | 3.1 | 3.4 |
| Königsh. [s] | (7d 8°/80%) | 18 | 17 | 18 | 12 | 20 | 18 |
| | (+2d NK) | 37 | 33 | 49 | 39 | 55 | 45 |
| | (+7d NK) | 59 | 55 | 60 | 48 | 70 | 64 |
| | (+14d NK) | 80 | 74 | 73 | 59 | 98 | 95 |
| Aspekt (8°/80%) | | matt | schön | schön | schön | matt | schön |
| Blushing | | 1 | 0 | 0 | 0 | 1 | 0 |
| Ring | | leicht | kein | kein | stark | mittel | kein |

| | | | | | | | |
|---|---|---|---|---|---|---|---|
| ¹ Gewichtsverhältnis zwischen N-Benzyl-1,2-ethandiamin und N,N'-Dibenzyl-1,2-ethandiamin | | | | | | | |

## Patentansprüche

1. Härter für Epoxidharze enthaltend N-Benzyl-1,2-ethandiamin und N,N'-Dibenzyl-1,2-ethandiamin in einem Gewichtsverhältnis im Bereich von 65/35 bis 95/5, **dadurch gekennzeichnet, dass** er eine Reaktionsmischung erhalten aus der reduktiven Alkylierung von 1,2-Ethandiamin mit Benzaldehyd und Wasserstoff, enthaltend N-Benzyl-1,2-ethandiamin und N,N'-Dibenzyl-1,2-ethandiamin in einem Gewichtsverhältnis im Bereich von 65/35 bis 95/5, insbesondere 70/30 bis 90/10, enthält.

2. Härter für Epoxidharze enthaltend N-Benzyl-1,2-ethandiamin und N,N'-Dibenzyl-1,2-ethandiamin in einem Gewichtsverhältnis im Bereich von 65/35 bis 95/5, **dadurch gekennzeichnet, dass** er mindestens ein weiteres Amin enthält und dass das weitere Amin ausgewählt ist aus der Gruppe bestehend aus 2,2(4),4-Trimethyl-1,6-hexandiamin, 1,2-, 1,3- oder 1,4-Diaminocyclohexan, 1,3-Bis(aminomethyl)cyclohexan, 1,4-Bis(aminomethyl)cyclohexan, Bis(4-aminocyclohexyl)methan, 1-Amino-3-aminomethyl-3,5,5-trimethylcyclohexan, 2(4)-Methyl-1,3-diaminocyclohexan, 1,3-Bis(aminomethyl)benzol, Polyoxypropylendiaminen mit einem mittlerem Molekulargewicht Mₙ im Bereich von 200 bis 500 g/mol, 3-(3-(Dimethylamino)propylamino)propylamin, Bis(6-aminohexyl)amin, Diethylentriamin, Triethylentetramin, Tetraethylenpentamin, Pentaethylenhexamin, Dipropylentriamin, N-(2-Aminoethyl)-1,3-propandiamin, N,N'-Bis(3-aminopropyl)ethylendiamin, Addukte dieser oder weiterer Polyamine mit Mono- oder Diepoxiden und Mannich-Basen.

3. Härter gemäss Anspruch 2, **dadurch gekennzeichnet, dass** das weitere Amin 1-Amino-3-aminomethyl-3,5,5-trimethylcyclohexan ist.

4. Härter gemäss Anspruch 2, **dadurch gekennzeichnet, dass** das weitere Amin 1,3-Bis(aminomethyl)benzol ist.

5. Härter gemäss Anspruch 2, **dadurch gekennzeichnet, dass** das weitere Amin Triethylentetramin, Tetraethylenpentamin, Pentaethylenhexamin oder N,N'-Bis(3-aminopropyl)ethylendiamin ist.

6. Härter gemäss Anspruch 2, **dadurch gekennzeichnet, dass** das weitere Amin 3-(3-(Dimethylamino)propylamino)propylamin ist.

7. Epoxidharz-Zusammensetzung umfassend
- eine Harz-Komponente enthaltend mindestens ein Epoxidharz und
- eine Härter-Komponente enthaltend einen Härter für Epoxidharze enthaltend N-Benzyl-1,2-ethandiamin und N,N'-Dibenzyl-1,2-ethandiamin in einem Gewichtsverhältnis im Bereich von 65/35 bis 95/5 oder den Härter gemäss einem der Ansprüche 2 bis 6.

8. Epoxidharz-Zusammensetzung gemäss Anspruch 7, **dadurch gekennzeichnet, dass** sie mindestens einen weiteren Bestandteil ausgewählt aus der Gruppe bestehend aus Verdünnern, Beschleunigern und Füllstoffen enthält.

9. Verwendung der Epoxidharz-Zusammensetzung gemäss einem der Ansprüche 7 oder 8 als Beschichtung, Primer, Klebstoff, Dichtstoff, Vergussmasse, Giessharz oder als Matrix für Faserverbundwerkstoffe.

10. Verfahren zum Beschichten umfassend die Schritte
(i) Vermischen der Komponenten der Epoxidharz-Zusammensetzung gemäss einem der Ansprüche 7 oder 8,
(ii) Applizieren der vermischten Zusammensetzung auf ein Substrat innerhalb der Topfzeit,
gefolgt von der Aushärtung der vermischten Zusammensetzung.

11. Verfahren zum Verkleben umfassend die Schritte
(i) Vermischen der Komponenten der Epoxidharz-Zusammensetzung gemäss einem der Ansprüche 7 oder 8,
(ii) Applizieren der vermischten Zusammensetzung innerhalb der Topfzeit,
- entweder auf mindestens eines der zu verklebenden Substrate und Fügen der Substrate zu einer Verklebung innerhalb der Offenzeit,
- oder in einen Hohlraum oder Spalt zwischen mehreren Substraten und gegebenenfalls Einfügen eines Ankers in den Hohlraum oder Spalt innerhalb der Offenzeit,
gefolgt von der Aushärtung der vermischten Zusammensetzung.

12. Artikel erhalten aus der Verwendung gemäss Anspruch 9 oder einem Verfahren gemäss einem der Ansprüche 10 oder 11.

## Claims

1. Curing agent for epoxy resins comprising N-benzylethane-1,2-diamine and N,N'-dibenzylethane-1,2-diamine in a weight ratio in the range from 65/35 to 95/5, **characterized in that** it comprises a reaction mixture obtained from the reductive alkylation of ethane-1,2-diamine with benzaldehyde and hydrogen, containing N-benzylethane-1,2-diamine and N,N'-dibenzylethane-1,2-diamine in a weight ratio in the range from 65/35 to 95/5, especially 70/30 to 90/10.

2. Curing agent for epoxy resins comprising N-benzylethane-1,2-diamine and N,N'-dibenzylethane-1,2-diamine in a weight ratio in the range from 65/35 to 95/5, **characterized in that** it contains at least one further amine and **in that** the further amine is selected from the group consisting of 2,2(4),4-trimethylhexane-1,6-diamine, 1,2-, 1,3- or 1,4-diaminocyclohexane, 1,3-bis(aminomethyl)cyclohexane, 1,4-bis(aminomethyl)cyclohexane, bis(4-aminocyclohexyl)methane, 1-amino-3-aminomethyl-3,5,5-trimethylcyclohexane, 2(4)-methyl-1,3-diaminocyclohexane, 1,3-bis(aminomethyl)benzene, polyoxy-propylenediamines having an average molecular weight Mn in the range from 200 to 500 g/mol, 3-(3-(dimethylamino)propylamino)propylamine, bis(6-aminohexyl)amine, diethylenetriamine, triethylenetetramine, tetraethylenepentamine, pentaethylenehexamine, dipropylenetriamine, N-(2-aminoethyl)-propane-1,3-diamine, N,N'-bis(3-aminopropyl)-ethylenediamine, adducts of these or further polyamines with mono- or diepoxides and Mannich bases.

3. Curing agent according to Claim 2, **characterized in that** the further amine is 1-amino-3-aminomethyl-3,5,5-trimethylcyclohexane.

4. Curing agent according to Claim 2, **characterized in that** the further amine is 1,3-bis(aminomethyl)-benzene.

5. Curing agent according to Claim 2, **characterized in that** the further amine is triethylenetetramine, tetraethylenepentamine, pentaethylenehexamine or N,N'-bis(3-aminopropyl)ethylenediamine.

6. Curing agent according to Claim 2, **characterized in that** the further amine is 3-(3-(dimethylamino)-propylamino)propylamine.

7. Epoxy resin composition comprising
- a resin component comprising at least one epoxy resin und
- a curing agent component comprising a curing agent for epoxy resins containing N-benzylethane-1,2-diamine and N,N'-dibenzylethane-1,2-diamine in a weight ratio in the range from 65/35 to 95/5 or the curing agent according to any of Claims 2 to 6.

8. Epoxy resin composition according to Claim 7, **characterized in that** it comprises at least one further constituent selected from the group consisting of thinners, accelerators and fillers.

9. Use of the epoxy resin composition according to either of Claims 7 and 8 as coating, primer, adhesive, sealant, encapsulating compound, casting resin or as matrix for fibre composite materials.

10. Method of coating, comprising the steps of
(i) mixing the components of the epoxy resin composition according to either of Claims 7 and 8,
(ii) applying the mixed composition to a substrate within the pot life,
followed by the curing of the mixed composition.

11. Method of bonding, comprising the steps of
(i) mixing the components of the epoxy resin composition according to either of Claims 7 and 8,
(ii) applying the mixed composition within the pot life,
- either to at least one of the substrates to be bonded and joining the substrates to form a bond within the open time,
- or in a cavity or gap between two or more substrates and optionally inserting an anchor into the cavity or gap within the open time,
followed by the curing of the mixed composition.

12. Article obtained from the use according to Claim 9 or a method according to either of Claims 10 and 11.

## Revendications

1. Durcisseur pour résines époxy contenant de la N-benzyl-1,2-éthanediamine et de la N,N'-dibenzyl-1,2-éthanediamine selon un rapport en poids dans la plage de 65/35 à 95/5, **caractérisé en ce qu'**il contient un mélange réactionnel obtenu par alkylation réductive de 1,2-éthanediamine avec du benzaldéhyde et de l'hydrogène, contenant de la N-benzyl-1,2-éthanediamine et de la N,N'-dibenzyl-1,2-éthanediamine selon un rapport en poids dans la plage de 65/35 à 95/5, en particulier de 70/30 à 90/10.

2. Durcisseur pour résines époxy contenant de la N-benzyl-1,2-éthanediamine et de la N,N'-dibenzyl-1,2-éthanediamine selon un rapport en poids dans la plage de 65/35 à 95/5, **caractérisé en ce qu'**il contient au moins une amine supplémentaire et **en ce que** l'amine supplémentaire est choisie dans le groupe consistant en la 2,2 (4),4-triméthyl-1,6-hexanediamine, le 1,2-, 1,3-ou 1,4-diaminocyclohexane, le 1,3-bis(aminométhyl)cyclohexane, le 1,4-bis(aminométhyl)cyclohexane, le bis(4-aminocyclohexyl)méthane, le 1-amino-3-aminométhyl-3,5,5-triméthylcyclohexane, le 2(4)-méthyl-1,3-diaminocyclohexane, le 1,3-bis(aminométhyl)benzène, les polyoxypropylènediamines ayant une masse moléculaire moyenne Mₙ dans la plage de 200 à 500 g/mole, la 3-(3-(diméthylamino)propylamino)propylamine, la bis(6-aminohexyl)amine, la diéthylènetriamine, la triéthylènetétramine, la tétraéthylènepentamine, la pentaéthylènehexamine, la dipropylènetriamine, la N-(2-aminoéthyl)-1,3-propanediamine, la N,N'-bis(3-aminopropyl)éthylènediamine, les produits d'addition de ces polyamines ou d'autres polyamines avec des mono- ou des diépoxydes et des bases de Mannich.

3. Durcisseur selon la revendication 2, **caractérisé en ce que** l'amine supplémentaire est le 1-amino-3-aminométhyl-3,5,5-triméthylcyclohexane.

4. Durcisseur selon la revendication 2, **caractérisé en ce que** l'amine supplémentaire est le 1,3-bis(aminométhyl)benzène.

5. Durcisseur selon la revendication 2, **caractérisé en ce que** l'amine supplémentaire est la triéthylènetétramine, la tétraéthylènepentamine, la pentaéthylènehexamine ou la N,N'-bis(3-aminopropyl)éthylènediamine.

6. Durcisseur selon la revendication 2, **caractérisé en ce que** l'amine supplémentaire est la 3-(3-(diméthylamino)propylamino)propylamine.

7. Composition de résines époxy, comprenant
- un composant résine contenant au moins une résine époxy et
- un composant durcisseur contenant un durcisseur pour résines époxy contenant de la N-benzyl-1,2-éthanediamine et de la N,N'-dibenzyl-1,2-éthanediamine selon un rapport en poids dans la plage de 65/35 à 95/5 ou le durcisseur selon l'une des revendications 2 à 6.

8. Composition de résines époxy selon la revendication 7, **caractérisée en ce qu'**elle contient au moins un constituant supplémentaire choisi dans le groupe consistant en les diluants, les accélérants et les charges.

9. Utilisation de la composition de résines époxy selon l'une des revendications 7 ou 8, en tant que revêtement, couche primaire, adhésif, matériau d'étanchéité, masse de scellement, résine de coulée, ou en tant que matrice pour matériaux composites de fibres.

10. Procédé de revêtement comprenant les étapes
(i) mélange des composants de la composition de résines époxy selon l'une des revendications 7 ou 8,
(ii) application de la composition mélangée sur un substrat pendant la vie en pot,
suivies du durcissement de la composition mélangée.

11. Procédé de collage comprenant les étapes
(i) mélange des composants de la composition de résines époxy selon l'une des revendications 7 ou 8,
(ii) application de la composition mélangée pendant la vie en pot,
- soit sur au moins l'un des substrats à coller, et assemblage des substrats pour obtenir un joint collé pendant le temps ouvert,
- soit dans un espace creux ou une fente entre plusieurs substrats et éventuellement insertion d'une ancre dans l'espace creux ou la fente pendant le temps ouvert,
suivies du durcissement de la composition mélangée.

12. Article obtenu par l'utilisation selon la revendication 9 ou d'un procédé selon l'une des revendications 10 ou 11.
